# EUROPEAN PATENT APPLICATION

(11) **EP 3 811 862 A1**
(43) Date of publication of application: **28.04.2021**
(21) Application number: 19220020.2
(22) Date of filing: 30.12.2019
(51) Int. Cl.: A61B 5/08, A61B 5/097, A61B 5/00, A61M 16/00, A61B 5/087, A61B 5/0205

(54) **VITAL PARAMETER MEASUREMENTS FOR LOW CARE PATIENTS**

(30) Priority: 22.10.2019 EP 19204732
(71) Applicant: Oxypoint NV, 2018 Antwerpen (BE)
(72) Inventor: HENDRICKX, Philip, 2600 Berchem (BE); VAN STEENKISTE, Cedric, 2547 Lint (BE); PINTENS, Niels, 2900 Schoten (BE); BORGONJON, Dirk, 2950 Kapellen (BE); VAN ROOST, Wouter, 1981 Hofstade (BE); HERMANS, Paul, 2660 Hoboken (BE)
(74) Representative: DenK iP bv

(57) **Abstract**

A system for measuring the vital parameters of low care patients is described. The system comprises a connecting element for a nasal cannula or breathing mask for providing a fluidic connection with the patient and a flow and/or pressure sensor in fluidic connection with the nasal cannula or breathing mask, for sensing, when the nasal cannula or breathing mask is connected to the system, at least a negative pressure signal. The system also comprises a processor configured for deriving, directly based on said at least a negative pressure signal, information related to the breathing cycle, and an output means configured for outputting at least one vital parameter of the patient, the outputting comprising outputting information related to the breathing cycle.

## Description

### Field of the invention

The invention relates to the medical field. More particularly, the present invention relates to systems and methods for measuring vital parameter for thus monitoring low care patients. Measuring the vital parameters includes at least information about the breathing cycle, using a pressure or flow measurement using a nasal cannula or breathing mask.

### Background of the invention

To monitor the general status of a patient, the nursing staff should measure the vital parameters for each patient at least 3 times a day. The 6 vital parameters are: temperature, blood pressure, breathing rhythm, heart rate, oxygen saturation and a consciousness score (in some more critical cases, urine and bowel movements are also monitored). It is on the basis of these measured values that, among other things, further therapy decisions are made. During the check-up, these parameters are often measured and written down manually, only to be archived in an electronic patient file. Measuring, registering and digitizing parameters is very time and labour intensive.

The measurement of the breath rhythm - expressed in number of breaths per minute (bpm) - is usually done in hospitals by counting the number of breaths of the patient for 20 seconds and afterwards multiplying the obtained value by 3 to a value per minute. However, this is inaccurate and also highly biased. During this snapshot, the parameters of the patient involved are after all influenced by the so-called white coat effect and deviate from the actual values, purely by the presence of the hospital staff in the patient room. Due to the cumbersome and inaccurate nature, this procedure is sometimes skipped among less critical patients if the nursing staff finds that they are doing well.

In the current situation there is a lack of therapy feedback, transparency and control (unless it concerns critical, high-care patients whose parameters are meticulously or even continuously monitored via expensive, cumbersome equipment). It is unknown what happens between 2 parameter rounds. In case of oxygen therapy delivery it is for instance unknown if the patient holds his nasal cannula. Does the patient sometimes breathe through the mouth? Has the patient been breathing harder, weaker, faster or slower? Have the breathing parameters remained stable? And so on. There is no certainty for the doctor that the prescribed dose has actually been administered. In addition, it is unknown whether the patient does take in oxygen when the caregiver leaves the room. Even at night and when the patient is asleep, it is not known what happens and whether the correct dose was taken. These are all examples considering oxygen therapy, similar examples can be given considering other forms of therapy where it is of interest to follow up the same vital parameters. Because there is no continuous follow-up possible for less critical patients, these people often do not receive optimal therapy between the measuring points. This has consequences for the well-being of the patient, the effectiveness of the treatment and ultimately the general length of stay in the hospital.

The use of gases in medical applications is widespread. One of the gases often administered to patients for medical reasons is oxygen. In view of the fact that administering oxygen is often essential for preventing damage to tissue, avoiding life-threatening situations or saving a patient from a life-threatening situation, hospitals distribute oxygen using a pipeline network up to the bed of nearly each patient. Furthermore, oxygen therapy is also widespread for homecare patients, making use of pressurised oxygen bottles and or oxygen concentrators. The conventional form of administering oxygen through a pipeline network is in a continuous manner and lacks any means for monitoring the therapy delivered. A flow meter can be set to the flow rate that the patient needs (1 I/min up to 15 I/min continuously or higher). This way the oxygen flows continuously from the source to the patient via a nasal cannula during inhaling and exhaling. In hospitals, the flow meter is plugged into the low-pressure oxygen socket (in Europe usually between 3.6 and 5.5 bars) on the wall behind the patient's bed. A moisturizer can be attached to the bottom side to prevent drying up of the nasal mucous membrane. Similarly at home, the flow meter is connected to the pressurised oxygen bottle, whereby typically a pressure reducer is introduced in between the flow meter and the pressurised oxygen bottle or is integrated in the bottle or the oxygen concentrator.

The lungs can only utilise the first phase of inhalation to exchange oxygen with the blood circulation. It is clear that oxygen can no longer be 'consumed' during the expiration phase (exhalation). However, during the last phase of inhalation too, only the large bronchial tube that does not participate in the diffusion process of oxygen is filled. Gas valves for the pulsating supply of medical gases are based on this principle: oxygen is purposefully administered during the first phase of inhalation by the patient. This way a maximum oxygen intake is achieved and the oxygen that is not used is minimised. This results in oxygen conserving without impacting the oxygen therapy. Various gas valves have been developed during the past 20 years which are based on the principle of discontinuous oxygen administration in order to increase the mobility of patients that use oxygen cylinders at home and/or to increase the autonomy of recipients. After all, the use of gas valves for mobile patients saves oxygen, which results in a lower consumption and consequently creating longer autonomy, i.e.3 to 5 times longer than the autonomy achieved with continuous administration.

Most gas valves are based on a regulating system in which nasal inhalation activates the gas valve. The underpressure activates and opens an oxygen valve through which an oxygen pulse (aka bolus) is generated. Detecting the inhalation and supplying the oxygen occurs by means of a nasal cannula. A nasal cannula can be a one-channel system (detection and supply occur through the same channel) or a two-channel system in which one channel is used for detecting the inhalation and the other channel is used for supplying the oxygen.

For most patients making use of medical gas therapy, especially those in hospitals, it is often required to monitor their vital parameters. Traditionally, typically up to six vital parameters are monitored to assess the patient amongst which pulse, respiratory rate, blood pressure and temperature. These parameters typically are collected a number of times per day, by nurses performing different measurements. This requires quite some time and puts a heavy burden on the nursing staff. Although for the monitoring of some vital parameters, there exist systems for more automated monitoring, the latter is not generally widespread, since it requires additional components and measuring/monitoring systems.

Also during home therapy, patients may be asked to monitor vital parameters by performing different measurements. Patients then typically note these down on a list, which is checked by a medical doctor when he visits the patient.

There is still room for improvement.

### Summary of the invention

It is an object of embodiments of the present invention to provide good methods and systems for monitoring vital parameters including at least information about the breathing cycle.

It is an advantage of at least some embodiments of the present invention that information regarding the breathing cycle and optionally also other vital parameters can be collected and displayed or outputted as data, for example towards the electronic patient family.

It is an advantage of at least some embodiments of the present invention that information regarding the breathing cycle and optionally also other vital parameters can be collected and displayed or outputted by a gas valve, which may be present anyway for delivery of medical gas therapy, thus allowing collection and display or output of such information with a reduced number of components or systems required.

It is an advantage of at least some embodiments of the present invention that information regarding the breathing cycle can be collected in an automatic manner, without nursing staff needing to perform additional task. The latter results in additional time being available for the nursing staff, since no additional time needs to be invested for registering of at least some of the vital functions. It is an advantage of at least some embodiments of the present invention that the workload of care-givers, e.g. nursing staff, can be substantially reduced.

It is an advantage of at least some embodiments of the present invention that medical gas, e.g. oxygen, use and medical gas, e.g. oxygen, therapy can be optimized. It is an advantage of at least some embodiments of the present invention that good or improved medical gas therapy administering can be performed.

It is an advantage of at least some embodiments of the present invention that administering of medical gas therapy can be optimized by making use of data information capturing and data analysis for one patient, for a group of patients e.g. suffering from similar conditions, for all patients in a hospital or even for different patients over different hospitals.

It is an advantage of embodiments of the present invention, that smart measuring devices are provided for use in the patient rooms, that are equipped with modern communication technology for enabling a link with the rapidly evolving ecosystem of wearables and sensors that are gradually entering the patient rooms. Making smart, connected measuring devices available in the patient rooms offers enormous opportunities. In some advantages, these devices combine smart connection to wearables and sensors with the possibility of controlling delivery of medical gasses, such as during oxygen therapy.

It is an advantage of embodiments of the present invention that systems and devices are provided that smart measuring devices and data collecting devices are provided and that are in direct data communication with a patient and associated software platform. It is an advantage of at least some embodiments that the smart measuring device can be combined with a device for providing for smart oxygen therapy. In some embodiments, measurements or data collection of one, more or all of the 6 vital parameters can be performed by the system. It is an advantage of at least some embodiments which combine the smart measuring device with medical gas therapy delivery that a further reduction of the amount of medical gas required for providing good therapy can be obtained. The built-in sensors make the trigger mechanism very sensitive. This optimizes use, ensures wider deployability of the devices, and thus results in even more comfort. Because on the one hand more patients can use the comfort mode (more patients per service and deployable in more services), and on the other hand the oxygen therapy can be phased out more quickly due to the improved follow-up, use of systems according to embodiments of the present invention will bring about an additional reduction in both oxygen consumption and consumption of additional aids such as humidifiers.

It is an advantage of embodiments of the present invention that automatic follow-up of patient parameters ensures peace of mind during nursing because the nursing staff knows that the patient always receives the correct dose, day and night. Furthermore, using embodiments according to the present invention, it becomes visible when the patient does not wear his nose cannula or when the patient is breathing. In addition, the automatic follow-up ensures that fewer manual measurements and interventions are used. Because the measurement results can also be logged automatically in the patient records, this reduces the general administrative workload.

It is an advantage of embodiments of the present invention that the accuracy of the measurements, such as the automatic measurement of the number of breaths per minute, ensures a better view of the patient's condition. Personalization and automatic follow-up result in a therapy tailored to the patient because it is adapted to the current need at any time. Events are also recorded between 2 nurses, which leads to improvements in individual care and therapy.

It is an advantage of embodiments of the present invention that a set of parameters can be measured at predetermined times, upon request or even continuously. Examples of technical parameters can be the delivered flow and the total consumption. In addition, it is for example also possible to measure and monitor patient-related parameters, such as the pressure caused by the patient's breathing cycle and the breathing frequency. It is an advantage that parameters can be logged automatically and can be automatically saved, e.g. in a storage device, or in the electronic patient records (EPD) via the software platform.

It is an advantage of at least some embodiments that the smart measuring device is able to link with neighbouring sensors such as wearables or other devices that measure other vital parameters. In this way, the parameters measured can be automatically integrated into the data streams. The data can be processed by increasingly smart algorithms to make the results available to hospital staff in various ways. This opens the door to tailor-made therapy: the patient's condition becomes relevant through the continuous measurement of parameters so that the therapy can be adjusted immediately. This adjustment can be suggested or implemented in real time in a further future (closed loop). Specifically, this adjustment may include an automatic decrease and increase in oxygen therapy as well as inducing changes in release mode (comfort vs. continuous) tailored to the effective need of the patient. Problems with people who breathe more gently and mouth-breathers can also be solved this way. Specific algorithms and programs are tailored to specific services (e.g., pneumology, cardiology, geriatrics, OR recovery), to certain diseases (e.g., COPD, heart failure, postoperative recovery), and to specific states of patients (e.g. during anesthesia, sleep or on the move in the kine space).

The present invention relates to a system for measuring at least one vital parameter, the system comprising a connecting element for a nasal cannula or breathing mask for providing a fluidic connection with patient, a flow and/or pressure sensor in fluidic connection with the nasal cannula or breathing mask, for sensing, when the nasal cannula or breathing mask is connected to the system, at least a negative pressure signal, a processor configured for deriving, directly based on said at least a negative pressure signal, information related to the breathing cycle, and an output means configured for outputting at least one vital parameter of the patient, the outputting comprising outputting information related to the breathing cycle. The output means may comprise a displaying means for displaying information of said at least one vital parameter of the patient. The displaying means may be a display for graphically or numerically displaying information. The displaying means may be a visual indication, such as for example a LED ring.

The output means may be configured for storing the at least one vital parameter of the patient in an electronic patient file, in a processing system or a memory.

The at least a negative pressure signal may be a negative pressure signal between 0.05mm H2O and 50mm H2O, for example between 0.05mm H20 and 10mm H2O in the nasal cannula; This may be used for triggering delivery of the medical gas.

Said deriving information related to the breathing cycle may comprise continuously deriving the information.

The flow and/or pressure sensor may be adapted for sensing independent of the delivery of the medical gas.

The processor may be configured for deriving breathing rate, a number of breaths per minute or a regularity.

The processor may be configured for deriving a pressure signal as function of time over at least one breath cycle.

The processor may be adapted for collecting, processing, storing or transmitting information regarding the breath cycle.

The system furthermore may comprise a port for receiving further patient information, such as temperature, oxygen saturation level in the blood, heart rate,....., from a patient monitoring/sensing device such as for example wearables and/or sensors.

The system furthermore may comprise a port for sharing breath cycle information and optionally other patient information with an electronic patient record.

The system furthermore may be adapted for coupling information of a breathing cycle to information regarding a patient's disease, a pathology or a health condition.

The system may be adapted for coupling information of a breathing cycle for patients of a certain group of patients and/or for patients performing a same activity.

The system furthermore may be adapted for predicting a breathing behaviour of a patient based on a patient's individual recorded previous breathing behaviour or based on previously recorded breathing cycle information of a group of patients.

The system furthermore may be adapted for suggesting an amendment of the medical gas therapy applied.

Predicting a breathing behaviour may comprise pro-actively adapting a medical gas therapy based on a patient's individual recorded previous breathing behaviour, or based on previously recorded breathing cycle information of a group of patients, or based on a change in a certain vital parameter such as for example a change in saturation level.

Predicting a breathing behaviour may comprise automatically adapting a medical gas therapy based on a patient's individual recorded previous breathing behaviour, or based on previously recorded breathing cycle information of a group of patients.

The system furthermore may be configured for checking whether the used therapy differs from a predetermined prescription.

The system may comprise further sensors or devices for deriving, measuring or determining further vital parameters.

In one aspect, the present invention also relates to a medical gas container or an oxygenator comprising a system as described above.

In another aspect, the present invention also relates to a software platform adapted for gathering information regarding at least one but optionally more vital parameters, wherein the software platform allows for receiving, storing, collecting and/or processing of data. The software platform may communicate with devices as described in the first aspect and/or with the electronic patient file.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

FIG 1 illustrates a front view of a system for delivering a medical gas according to the present invention.
FIG. 2 illustrates a side view of a system for delivering a medical gas according to the present invention.
FIG. 3 illustrates how the airway gets blocked interrupting the air flow during the breathing cycle, as known from prior art (available on the internet).
FIG. 4 illustrates how PAP devices induce a positive pressure to keep the airway open at all time so it doesn't get blocked, as known from prior art (available on the internet).

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect, the present invention relates to a system for measuring at least one vital parameter.

The system performs the function of measuring a vital parameter comprising at least information regarding the breathing cycle. Such information may for example be a breath rate, but embodiments are not limited thereto and also a regularity of breathing, the quality of breathing and other related parameters may be collected. According to some embodiments of the present invention, the system can be also used just for measuring functions and thus without delivering any medical gas.

According to embodiments of the present invention, the system comprises a connecting element for connecting a nasal cannula or breathing mask to the system, for providing a fluidic connection to the patient. The connecting element may be referred to as a lead-through (also referred to as an outlet) for connecting the system to lead the generated gas flow to the patient. A classic example of such a system is a nasal cannula. Lead-through 150 can be a one-channel or a two-channel lead-through (e.g., for connecting a one-channel or two-channel nasal cannula). A two-channel lead-through has two channels in which one channel is used for supplying the medical gas and the other channel is used for detecting the nasal inhalation at a specific moment. The lead-through can have a fitting connection for nasal cannulas available on the market for supplying medical gases to patients. Lead-through can have a specific geometry in a specific embodiment in order to correspond to a specific geometry of the system's connection that supplies the generated gas flow to the patient (e.g., a nasal cannula with a connection of a specific geometry). This will prevent a situation in which the system supplying the generated gas flow to the patient is connected by mistake to the wrong pipeline (e.g., a compressed air pipeline). In some embodiments, the system can be adjusted to operate with a two-channel nasal cannula and the system used for detecting the nasal inhalation at a specific time can be used to initiate the gas pulse. In other words, the pulse operation can be regulated on the basis of the detected signal. In other embodiments, the valve is adjusted to operate with a one-channel nasal cannula and the pulse operation is initiated by creating underpressure in the one channel. Similarly, the system may be adapted for operation with a breathing mask, rather than a nasal cannula.
According to embodiments of the present invention, the system furthermore comprises a flow and/or pressure sensor in fluidic connection with the nasal cannula or breathing mask, for sensing the nasal or mouth inhalation as described above. Typically, this inhalation is sensed, when the nasal cannula or breathing mask is connected to the system, by at least a negative pressure signal. It is to be noted that aside the negative pressure signal, also positive pressure signals may be detected. In at least some embodiments, a full recording to the pressure during the breathing cycle is performed. Whereas reference is made to the pressure, alternatively also the flow of the gas may be recorded. Embodiments of the current invention are suitable preferably for patients, e.g. persons or animals, with spontaneous breathing.
The flow and/or pressure sensor typically may correspond with features of electronic systems for delivery of medical gasses allowing on demand delivery of medical gas. Such systems do not continuously provide medical gas, but provide medical gas during the proper moment in time of the breathing cycle when the patient optimally benefits from the medical gas delivery. Such systems typically are developed to conserve medical gasses. The type of flow and/or pressure sensors that are used may be any type of flow and/or pressure sensor having sufficient sensitivity to accurately measure the breathing cycle of the patient. In some embodiments, the sensitivity should be sufficiently good to detect a negative pressure signal in the range between 0.05mm H2O and 50mm H2O, e.g. between 0.1mm H2O and 10mm H2O, for triggering the delivery of medical gas when such a negative pressure is triggered. The pressure sensor or flow sensor may be adapted for deriving a pressure signal at predetermined moments in time, during at least one full breathing cycle, during a plurality of breathing cycles, etc. It may be adapted for deriving a pressure signal as function of time during at least one full breathing cycle or during a plurality of breathing cycles. The components of the system adapted for delivery of gas, on demand or continuous, may in some embodiments be equal to or similar to the system as described in international patent application PCT/IB2012/05329.
The system furthermore comprises a processor configured for deriving information related to the breathing cycle, directly based on at least a negative pressure signal. Where in embodiments of the present invention reference is made to directly based on at least a negative pressure signal, reference is made to the fact that there is a direct link between the pressure signal sensed and the information regarding the breathing cycle. The latter is advantageous since e.g. whether or not a pulse of medical gas is created is irrelevant for determining the information regarding the breathing cycle. In some embodiments, the information of the breathing cycle may be determined based on a predetermined value of negative pressure sensed, based on a full pressure signal sensed over the full breathing cycle, .... The information regarding the breathing cycle may be for example a breathing rate, a regularity or irregularity in the breathing cycle or in the breathing rate, a number of breaths per minute, an intensity of breathing, etc. The system may be adapted for continuously deriving the information regarding the breathing cycle or for deriving such information at predetermined moments in time, at a fixed frequency, when triggered by medical or nursing staff, etc.

According to embodiments of the present invention, the system furthermore comprises an output means configured for outputting at least one vital parameter of the patient, the outputting comprising outputting information related to the breathing cycle. Such an output means may in some embodiments be an output device locally to the system, such as for example a display, a visual indication means, such as for example a LED indication, or an auditive indication means or a combination thereof. In some embodiments, the vital parameter may be displayed in a numerical or graphical manner. In some embodiments, a value or range of values may be indicated using a colour based graphical representation. Aside from, alternative to or in combination with the one or more vital parameters, also information regarding the applied medical gas therapy may be indicated in one of the above indicated manners.

In some embodiments, the output means may be an output port for outputting data to a memory, an electronic patient file, a processing means, a general data processing system for processing big data, etc. The output port may be adapted for outputting data to an external device positioned away from the system. The system therefore may have a transmitting means for transmitting the output data to an externally positioned device. Transmitting means for transmitting data are known to the person skilled in the art. They may rely on wired or wireless transmission. They may make use of conventional protocols or specific designed protocols. Typical protocols that may be applied are internet protocols, Bluetooth protocol, file transfer protocols, TCP and UDP-based protocols, through web services, etc. One particular example, embodiments of the present invention not being limited thereto, is communication according to a Bluetooth 4 protocol.

More generally, the device typically may be in direct connection with the patient on the one hand and the caregiver on the other. The communication with the patient and/or the caregiver may use any suitable communication platform such as for example Bluetooth. The data communication between the device and the software platform can make use of a same or a different protocol. To be able to monitor patient parameters in real time (and to be able to receive and carry out suggestive adjustments remotely), the device typically will collect the measured or estimated data. Since the data concerned are medical patient related data, the protocol used may be such that the data is anonymised, aggregated or well protected during transmission, so that the required confidentiality can be guaranteed.
The transmission of the data from the system may in some embodiments be performed only after a while. In order to store the data prior to transmission, one or more memory elements may be present in the system for controlling delivery of medical gasses.

The system may in some embodiments be adapted for collecting, aside from vital parameters that can be derived based on measurements performed by the system itself, further data measured or collected by devices in the neighbourhood of the system for controlling delivery of medical gasses. For example, the system for controlling delivery of medical gasses may be equipped for collecting temperature data from a temperature monitoring device, heart rate from a heart rate monitor, oxygen level related data from an oxygen level monitoring device, blood pressure information from a blood pressure device, or combination of these data from plural vital parameter monitoring devices etc. Data may be collected from wearables. Such data, once received in the system for controlling delivery of medical gasses, may be stored separately or in combination with other information such that for example different parameters that measured at the same or approximately the same time are grouped, and may be either displayed in any suitable manner or transmitted to an external device. Aside from vital parameters, also information regarding the patient, information regarding the pathology as well as information regarding the medical gas therapy that is applied may be collected, processed, stored and/or transmitted by the system.

In some embodiments, the system may be adapted for collecting information from cameras or systems allowing measurement of parameters such as vital parameters based on light/radiation signals. Examples of such systems, embodiments of the present invention not being limited thereto, are for example as described in US2014/0243649 A1, US2011/0054277 A1 or US2015/0105670 A1. Examples of parameters that can be obtained are parameters based on breathing, such as breathing frequency, oxygen saturation in the blood, body temperature, etc.

According to at least some embodiments wherein the system collects additional information from wearables or other information collection systems, the information regarding vital parameters obtained through the pressure measurements as described above may be used as validation information, calibration information or cross-check for the information obtained from wearables or other information collection systems, or vice versa.

According to at least some embodiments, the system is configured for sending the at least one vital parameter to the electronic patient file. Such data may be sent at predetermined moments in time, at a fixed frequency, continuously, upon trespassing certain predetermined values or upon request of the medical or nursing staff. The latter may be performed as well in a hospital environment as in a home environment. The latter advantageously may allow a medical doctor to have faster access to this medical information compared to the situation where the doctor only can access the information based on notes taken by the patient or nurse or information stored locally on the system.

In some embodiments, the system furthermore may be adapted for delivering a medical gas to a patient. The system may for example be a system for delivering oxygen therapy to a patient, although embodiments of the present invention are not limited thereto. The system may be suitable for use in hospitals as well as for use at home. The system may be referred to as a gas valve for controlling the delivery of medical gas, but it will be clear from the previous description that such features are combined with the function of monitoring at least information regarding the breathing cycle. Such information may for example be a breath rate, but embodiments are not limited thereto and also a regularity of breathing, the quality of breathing and other related parameters may be collected. According to some embodiments of the present invention, the system can be also used just for measuring functions and thus without delivering any medical gas.

Systems according to such embodiments typically may include a connection component to connect the system to an external supply. Such an external supply can be an external supply network for medical gases at a pressure lower than 50 bars (e.g., 10 bars or lower), as typically will be present in a hospital. Alternatively, such a connection component can be adjusted to connect to a gas canister or gas cylinder at a pressure higher than 50 bars (e.g., approx. 200 bars) or a concentrator. Such cylinders enable an uninterrupted supply of oxygen during oxygen therapy in case the patient is moved from one department to another or allow for home medical gas therapy. The disadvantage is that consumption must be monitored so that the cylinders are replaced on time. In embodiments according to the present invention, the connection component can be adjusted to correspond with typical connections in the supply network. This can be a connection of a shape laid down in the law, for example, according to DIN norms, CARBA norms, BOC norms or AFNOR norms, Parcodex, AGA (Swedish standard), Medap, etc. etc.. It should be clear that the precise type of an installed connection is not limiting for embodiments of the present invention. For example, such an external supply network can be a network for distributing medical gases in a hospital and can supply such a medical gas at a pressure which is for example in Europe determined in the range of 3.3 bars up to 5.5 bars or similar. A commonly found gas supply network, which one finds in hospitals, is the facility of oxygen connections for patients in each room. In a specific embodiment of the present invention, the connection component is equipped with a special geometry that fits a connection to a specific gas supply network eliminating any errors between different gas supply networks.

In one aspect, the present invention also relates to collecting, processing, storing and/or transmitting of details regarding the medical gas therapy rather than information regarding vital parameters or in addition thereto. The latter may allow for example to accurately monitor the administering of medical gas for a particular patient, a group of patients, a certain division of a medical hospital, a hospital as whole, for patients having been diagnosed with a same pathology, etc. It may allow for deriving a particular medical gas therapy applied. In some embodiments it may allow for suggesting a particular medical gas therapy for a patient in the future to the medical staff. In some embodiments it may be used for automatically adjusting the medical gas therapy used for a patient. Such suggestions or adjustments may be based on data processing of the collected, processed, stored or transmitted data, e.g. allowing data processing for a group of patients having e.g. the same pathology, optionally dependent on specific patient characteristics such as gender, physical parameters, or physical conditions that can evolve during the stay etc. Such data processing could for example be based on predetermined algorithms, on neural network processing, etc.

In some embodiments, the system can be used for collecting information relating to localisation of devices or persons. Each of the devices may be identifiable in a communication network, such as a wireless network and may be linked to a position in this network. Such a wireless network may be based on every suitable protocol, such as for example Bluetooth or Wifi. Being in interaction with each other, the devices can form a mesh in which other devices can be positioned. The network may be a separate and/or private network, such that it is compliant with the required patient information safety requirements. Aside from the data collection regarding the gas therapy or gas logistics, also other parameters may be collected, such as for example a location of all Bluetooth, Wifi or other suitable protocol activated devices or persons that have been equipped with tags or integrated Bluetooth, Wifi or other suitable protocol technology. For instance persons or devices that move in the mesh could be localised.

In some embodiments, a system and method for collecting information relating to gas therapy or to gas logistics. The system may be based on gas information gathering in pressurized gas bottles. The gas information gathering may be based on data collection from a plurality of pressurized gas bottles. A data collection system, e.g. a data collection system as described above, may be integrated in each of the pressurized gas bottles or connectable to it. The latter may for example be obtained by an integrated gas valve system being present in the pressurized gas bottles or a gas valve system connected thereto, whereby the gas valve system comprises gas therapy or gas logistics data collection means. Each of the bottles may be identifiable in a communication network, such as a wireless network. Such a wireless network may be based on every suitable protocol, such as for example Bluetooth or Wifi. The network may be a separate and/or private network, such that it is compliant with the required patient information safety requirements. Aside from the data collection regarding the gas therapy or gas logistics, also other parameters may be collected, such as for example a location of all Bluetooth activated devices or persons through tags or integrated Bluetooth technology. For instance persons or pressurized gas bottles or objects that move in the mesh could be localised.

With mesh reference may be made to rich interconnection among devices or nodes. In a wireless mesh network, the data collection device may act as a mesh client, a mesh router or a gateway. A mesh may be a static mesh, whereby fixed data collecting devices are used, e.g. mounted to the wall in a hospital. Alternatively, such a mesh can also be dynamic, e.g. because data collecting devices are additionally coupled or decoupled during use. In yet another alternative, the mesh can be dynamic in that the different data collecting devices may be moveable and consequently the mesh changes continuously.

In one aspect, embodiments of the present invention can be used for gathering positional information regarding persons, gas bottles or other objects. The information gathered from the different nodes may be combined with a map so as to derive positional information. In some embodiments, some fixed reference may be used to refer to a map. In other embodiments, the information is combined with a map, so as to more easily allow meaningful localisation.

In some embodiments, the output means may be adapted for sending collected, processed, stored or transmitted data on the one hand directly to the electronic patient file and on the other hand, personalised or anonymised data to a data processor for processing collective data from numerous patients. Such a data processor may be programmed for deriving information of the vital parameters and/or the medical gas therapy if applied and/or a combination thereof for determining predetermined rules for suggesting or controlling certain processes or therapies, e.g. for suggesting, adjusting or controlling the medical gas therapy to be applied for a patient, e.g. for a patient with a certain pathology. It may furthermore be adapted for predicting a better medical gas therapy during further steps in the process. Alternatively or in addition thereto, sending the data to the data processor may also allow to compare it with earlier derived predetermined rules for suggesting, adjusting or controlling certain processor or therapies for that specific patient. For example, a suggestion may be sent to the electronic patient file for the medical doctor to be assisted in his/her decision or it may be sent to the system for controlling the delivery of medical gas to be displayed as a suggestion for medical gas therapy. Alternatively an automated adjustment may be sent to the system for controlling the delivery of medical gas such that the delivery of medical gas is automatically adjusted in line with predetermined rules.

Whereas the above embodiments have been described with reference to a patient pathology, the embodiments can also be related to a person's health condition or a patient's disease. Whereas some embodiments have been described with reference to a group of patients having the same pathology, embodiments equally can be applied to patients or people performing a same activity or using the same drugs or to compare the effect of drugs used and which are the most effective on the measured parameters.

According some embodiments, the system also may be adapted for predicting a breathing. behaviour. The processor of the system thus may be configured for predicting a breathing behaviour and optionally pro-actively adapting a medical gas therapy based on a patient's individual recorded previous breathing behaviour, or based on previously recorded breathing cycle information of a group of patients. Alternatively the medical gas therapy may be suggested to the medical staff rather than automatically adapted.

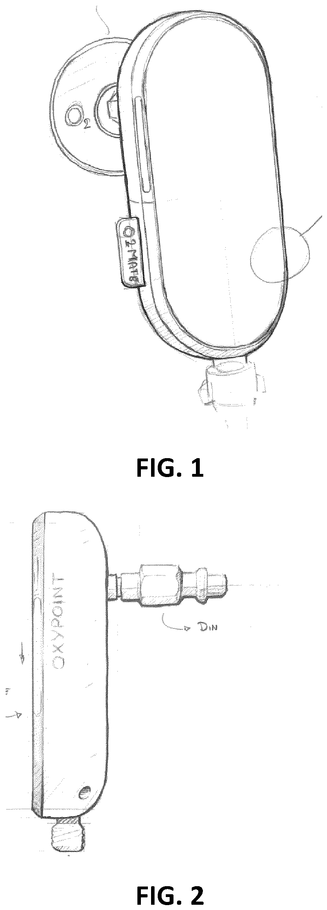

By way of illustration, embodiments of the present invention not being limited thereto, an example of a system is shown. The system shown is provided with a double display system. Centrally at the front there is a display, which can be used for displaying numerically or graphically parameters of the medical gas delivery or of the vital parameter. At the side an indication of a vital parameter or of the medical gas delivery, e.g. the type of delivery mode, can be given. Such indicators can make use of colour, use of a light signal circulating at the edge of the device, the speed of such travelling being indicative of a parameter, etc. A front view is shown in FIG. 1, whereas a side view is shown in FIG. 2.

In one aspect, the present invention relates to a software platform adapted for gathering information regarding at least one but optionally more vital parameters, wherein the software platform allows for receiving, storing, collecting and/or processing of data. The software platform may communicate with devices as described in the first aspect and/or with the electronic patient file. Further features and advantages may be as indicated in the other aspects. The software platform may be adapted for analysing conditions of patients and for adjusting treatments.

Within the knowledge platform, data concerning the breathing cycle and optionally also other vital parameters can be used as an indicator for the evolution and follow-up of many therapies and treatments. The breathing frequency can be measured continuously and very accurately. The data can be converted into a curve and can be combined with the data from the other 5 vital parameters so that everything can be viewed in one overview. This opens up interesting possibilities such as, for example, monitoring the effect of certain medication; a useful method in clinical studies by pharmaceutical companies.

In one example, according to an embodiment of the present invention, a program that helps set up and adjust oxygen therapy (start-up function) is described. When a new patient comes in and the need to administer extra oxygen is determined, titration is performed to determine the correct dose. In this way it is initially determined which dose is prescribed. This expresses itself in a number of liters per minute O2, for example 2 I / min. If there is a comfort mode, the method of administration must also be chosen: comfort or continuous. This is done by setting the comfort mode to the desired flow and looking and listening to see if the patient is able to trigger the comfort mode. After visual and auditory check, it is decided to save the setting or to switch to continuous mode. This is a fairly cumbersome and not objective procedure. Afterwards, saturation is regularly monitored, and at least 3 times a day, to adjust the dose as the patient's (health) condition evolves. Often on the basis of such a punctual measurement it is decided to give an extra day for example 2 I / min in comfort or continuously. An interesting specific functionality could be one in which the device helps care providers to make the right choice of flow rate and method of administration (continuous or comfort) based on the measurement data. For patients who have been present for some time, the interim measurements can also be used to adjust the patient efficiently during the nursing round.
In another example, according to an embodiment of the present invention, a program to monitor the night (night function) Healthcare providers are currently unsure of what happens during the night. Unless they are very critical patients who are continuously monitored such as at a high care, CCU, night surveillance or medium care service for example. But also for the low care departments it is useful to know whether the patient has been breathing regularly and whether he is not desaturating at night. That is why it seems useful to us to make the data generated during the night available to the health care providers who do their morning round, so that they themselves can be reassured and in turn they can reassure patients. There is some cold-water fear to set comfort mode at night, with such an application this should bring enough peace of mind to be able to confidently go for it, but this is equally relevant for a continuous setting. After all, when a patient starts snoring, breathing through the mouth, or accidentally taking off his nose glasses, he no longer takes the same dose in continuous.

In a third example, a soft, mouth respiration and nasal goggles detection program (breath variation function), as can be used in an embodiment of the present invention is disclosed. Patients often take their nose glasses off because of irritation caused by the dehydration of the nose and airways. Oxygen therapy is uncomfortable, leads to crusting, nose bleeds and in some cases headaches. Other patients will unknowingly breathe through the mouth or breathe softer. And then there are periods of sleep or carelessness in which the nose glasses shift or come loose. Because the breathing pressure is continuously monitored in some embodiments, it becomes possible to detect and respond to such events. An application that reminds the mouth breathers well by breathing the nose by, for example, releasing a number of puffs one after the other is disclosed. This series of puffs is recognized by the patient as a signal to breathe properly through the nose. Another application can be aimed at a signal function for the nursing staff in order to inform them of events that they can take into account.

In some embodiments, a full real-time personalization and automation of the medical gas delivery in the long term, whereby direct (closed-loop) therapy adjustments can be made based on permanent parameter monitoring is provided. For example, through a self-learning algorithm and pattern recognition, the system may get to know the patient's breathing pattern (and all relevant other parameters) during therapy in order to become more accurate with the outliers. In this way one can respond to the current events / therapy needs in a well-founded and real-time manner and in this way (for the first time in the history of oxygen therapy) one delivers a therapy fully tailored to the patient, which opens the door to future applications. Some examples thereof are shown below.
In a first example, a program for automatic reduction of medical gas therapy is described. After surgery, patients often receive a high dose of oxygen that may be phased out, first in the OR recovery service, and in some also (whether or not via a medium care service) in the low care post-operative departments, until the dose finally reaches zero is being brought. This has been a very labour-intensive task for nursing to date and often takes longer than is strictly necessary. During a parameter round with the doctor, it is regularly decided to hold a patient for an extra day or 2 days at a certain oxygen flow rate before attempting to reduce or switch to a lower flow. This means that in practice sometimes more days are used than strictly necessary. A program that helps automate this reduction on the basis of continuously measured parameters seems interesting to us. A difficulty and additional challenge is how this process can be improved before the closed loop options can be exempted.

In a second example, a program for automatic build-up of oxygen is described. During exercise, the kinesist will always calculate the corresponding fraction of inspired oxygen (FiO2) based on the breathing frequency and the supplied flow rate and, as the effort increases, the oxygen flow also increases. This is not a very accurate approach and very labour-intensive. A program that helps the physiotherapist build up oxygen delivery by giving on-screen indications or suggestions for adjusting the flow rate, can significantly improve therapy and workload. In a later phase the amount of oxygen can be (semi-) automatically increased based on set thresholds and the measured parameters within pre-set limits.

In a third example, a program for COPD patients (COPD function) is described. COPD patients who have only just been diagnosed and are receiving oxygen for the first time react completely differently to the comfort mode than COPD patients who have been receiving oxygen for a long time and already have certain stuck habits that are difficult to break. A program that helps to choose the correct delivery is interesting for both groups. From a certain stage of the disorder, these patients are admitted to the hospital on a regular basis, often coming in in the first days in a panic with a very annoying feeling of discomfort. These patients often exhibit accelerated breathing and feel anxious. They have no ear for the benefits of comfort fashion. and want oxygen as quickly as possible because they feel they will choke differently. For these patients it is currently recommended to first give oxygen continuously for 24 or 48 hours and then, when the feeling of distress has subsided, try to switch to comfort mode. The program for COPD patients could indicate when the optimum time has come to switch to comfort mode. Too high a release of oxygen is also dangerous for these patients because they can exhibit hypercapnia, with the risk of carbon anesthesia, which reduces the respiratory reflex and eventually disappears. This program could take this into account and, for example, provide a possibility that the patient cannot make adjustments to his own therapy, or at least register the changes and make them available to health care providers.

An interesting aspect of the constructed algorithms is that they can be reused intelligently for various applications. The program with which the correct mode can be selected is good for the start-up of any patient as well as for adjusting during the regular parameter rounds in the nursing units and can serve as a basis for the program for the night and even for those of the COPD patients. for example. The program for automatic collection and increase of oxygen therapy is useful for both a kine room and in an OK recovery or post-operative setting. It is also possible for the first time to combine the output of the oxygen dosing valve and the flow sensor. This allows us for the first time to manipulate the oxygen delivery curve. For example, one plans to optimize the curve in terms of noise production and the perceived comfort level of the patient through experiments. If we then also include the bpm trend analysis in the course, the volume of oxygen delivered in comfort mode can be adjusted to the respiratory rate of the patient. In this way it is possible to realize an oxygen delivery with a constant minute volume. This function ensures that the patient receives the exact prescribed dose of oxygen as a function of time. When the patient's breathing speeds up or slows down, the device can respond to this by increasing or decreasing the volume of oxygen delivered per breath. The delivery amount will therefore always adjust on the basis of the calculated bpm value.

The present invention also relates to a system for use in sleep apnoea. Sleep apnoea is a sleeping disorder where a person has pauses in breathing or periods of shallow breathing during the sleep. Such pauses can last for a few seconds to a few minutes. Because the disorder disrupts normal sleep, those affected may experience sleepiness or feel tired during the day. Sleep apnea may be either obstructive sleep apnea (OSA) in which breathing is interrupted by a blockage of air flow, central sleep apnea (CSA) in which regular unconscious breath simply stops, or a combination of the two. Obstructive (OSA) is the most common form. Treatment may include lifestyle changes, mouthpieces, breathing devices, and surgery. Breathing devices include the use of a CPAP machine. Positive airway pressure (PAP) is a mode of respiratory ventilation used in the treatment of sleep apnea. CPAP works by blowing air into the throat via a mask, subtly increasing air pressure in the throat and preventing the airway from narrowing. It's important that the seal over your airways is tight enough to allow for continuous air pressure from the CPAP device.

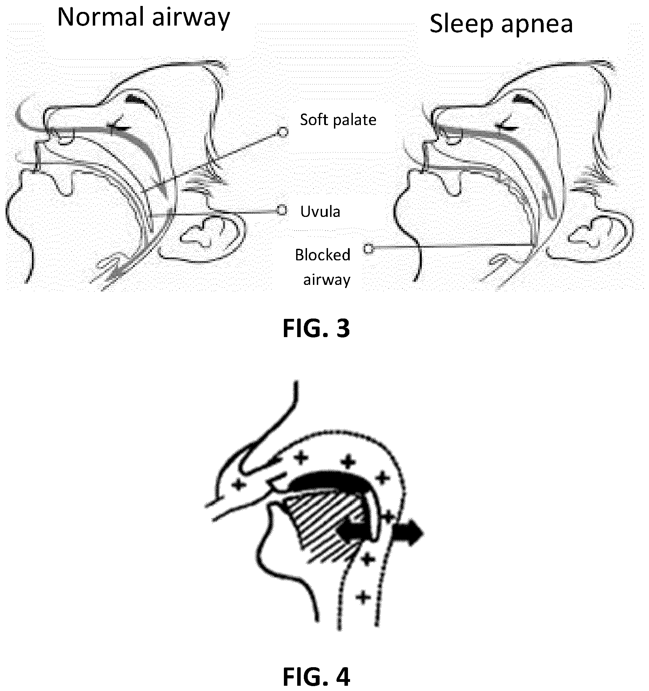

FIG. 3 illustrates how the airway gets blocked interrupting the air flow during the breathing cycle. FIG. 4 illustrates how PAP devices induce a positive pressure to keep the airway open at all time so it doesn't get blocked. This figure shows a device with a nose mask, increasing the pressure through the nose as to keep the throat (and the access to the lungs) open at all times. Similarly, a mask can be mounted on the face and connected to a compressor to make positive pressure and impose it to the patient's airway. Typically the CPAP devices are putting pressure continuously to the airways, and thus also when the airway is perfectly open and for certain patients there might be no need to intervene. These assemblies are quite intrusive, often also noisy, awkward to put on for every night and inconvenient for the users, both for the patient and his/her partner.

According to one aspect, the present invention also relates to a system and method for use in diagnosis of sleeping disorders such as but not restricted to snorring or sleep apnoea. A system according to embodiments of the present aspect comprises a system for gathering at least information regarding the breathing cycle whereby automatically the pressure or flow differences in breathing of the user are sensed, when the system is connected tot he patient via a nasal canula. The sensed data is used for detecting events of sleep apnoea such as pauses in breathing or periods of shallow breathing during the sleep to be able to help forming a right diagnosis for the specific patient. These devices can be used in a hospital or also at home.

According to one aspect, the present invention also relates to a system and method for use in sleep apnoea. A system according to embodiments of the present aspect comprises a system for gathering at least information regarding the breathing cycle whereby automatically the pressure or flow differences in breathing of the user are sensed, when the system is connected to the patient via a nasal canula. The sensed data is used for improving positive airway pressure delivery to patients. In a first embodiment this can be done by controlling a compressor or by controlling a CPAP device. In one embodiment, both the system for gathering information as well as a compressor or CPAP device are connected to the patient. The system for gathering information may be for example a system as described in one of the above apects. In some embodiments, both an information gathering means and a compressor or CPAP device are connected to the patient. The information gathering system and the compressor or CPAP device in such embodiments are interconnected. Communication between the information gathering system on the one hand and the compressor or CPAP device on the other hand could for example be through a wired or a non-wired communication system. In one particular example, communication may be performed through blue tooth or wifi. The idea is to regulate the pressure by the compressor or CPAP device with the pressure signals received from the information gathering system and thus to only produce pressure to be applied to the user when needed. For instance when PAP devices are being used against snorring, it has no use to continuously put pressure on airways that are free and well opened, only when an event of narrowing or closing airways is imminent or already happening for a certain amount of time and can cause snorring or in case of an imminent or actual system.

It is to be noted that different methods are known for applying pressure, e.g. some through the nose, some through the mouth. The present invention is not limited by the exact way of how the pressure is applied. Embodiments are envisaged using mouth masks, nose masks or a combination thereof. Combination with sensing pressure changes through the same mask that delivers pressurised air are envisaged but also configurations where the mask is only used for delivering the pressure while a nasal cannula (or other means) is used for sensing the pressure difference. In further alternatives, even an implied negative pressure may be used in the airway at the height of the mouth so that the passage through the nose and troat keeps open. In one embodiment, the system thus also may be implemented as an information gathering system mounted on a vacuum or pump outlet instead of a pressured air or gas output, because there it is possible to implement a negative pressure. In between sessions of implementing negative pressure, the device would again only measure, and thus not intervene in the natural breathing cycle, wich is more convenient fort he patients that tries to get sleep.

In still other embodiments, the system for gathering information may be connected to a gas supply system, or be the gas supply system itself, so that a gas supply in a certain way can help the patient to adapt his breathing. In a particular embodiment a burst of gas can be delivered to achieve a change in sleep position or a change the way of breathing of the patient to resolve a obstruction or shallow breathing event.

In some embodiments, the system may be adapted to measure the breathing cycle and to adapt the pressure released to the airway of the patient to synchronise it with the breathing cycle. This can be done with pressure sensors incorporated in the devices or with other technologies, such as sound capting devices or other sensors.

It is an advantage of embodiments according to the present invention that measurements can be performed all the time while pressure or underpressure is only applied when it is needed. It is an advantage of embodiments of the present invention that the delivery of pressure and the sensing can be done through different channels (e.g. pressure can be delivered through a mouth mask and breathing information is measured through the nasal cannula). This gives precise information, and a less disturbed signal than the one within the pressure delivering system itself, in which the measured pressure must go through the mask that is delivering the pressure itself.

## Claims

1. A system for measuring at least one vital parameter of a patient, the system comprising
- a connecting element for a nasal cannula or breathing mask for providing a fluidic connection with patient
- a flow and/or pressure sensor in fluidic connection with the nasal cannula or breathing mask, for sensing, when the nasal cannula or breathing mask is connected to the system, at least a negative pressure signal
- a processor configured for deriving, directly based on said at least a negative pressure signal, information related to the breathing cycle, and
- an output means integrated in the system and configured for outputting information regarding at least one vital parameter of the patient comprising information related to the breathing cycle.

2. A system according to claim 1, wherein the output means comprises a display, a visual means or an auditive means for providing information of said at least one vital parameter of the patient.

3. A system according to any of claims 1 or 2, wherein the output means may be configured for storing the at least one vital parameter of the patient or for transmitting the at least one vital parameter of the patient towards an external processing system, an external memory, a big data processing system and/or an electronic patient file.

4. A system according to any of the previous claims, wherein said deriving information related to the breathing cycle comprises continuously deriving the information.

5. A system according to any of the previous claims, wherein the processor is configured for deriving breathing rate, a number of breaths per minute or a regularity and/or wherein the processor is configured for deriving a pressure signal as function of time over at least one breath cycle or wherein the processor is adapted for collecting, storing, processing or transmitting information regarding the breath cycle.

6. A system according to any of the previous claims, wherein the system furthermore comprises a port for receiving further patient information, such as one or more of oxygen level in the blood, heart rate, blood pressure, temperature, ....., from a patient monitoring/sensing device such as for example wearables or sensors.

7. A system according to claim 6, wherein the system furthermore comprises a port for sharing breath cycle information and optionally other patient information with an electronic patient record and/or wherein the system is adapted for coupling information of a breathing cycle to information regarding a patient's disease, a pathology or a health condition.

8. A system according to claim 7, wherein he system is adapted for coupling information of a breathing cycle for patients of a certain group of patients and/or for patients performing a same activity.

9. A system according to any of the previous claims, wherein the system furthermore is adapted for predicting a breathing behaviour of a patient based on a patient's individual recorded previous breathing behaviour or based on previously recorded breathing cycle information of a group of patients.

10. A system according to any of the previous claims, wherein the system furthermore is adapted for delivering medical gas to a patient, or
wherein the flow and/or pressure sensor is adapted for sensing independent of delivery of medical gas to a patient via the system.

11. A system according to claim 10, wherein the at least a negative pressure signal is a negative pressure signal between 0.05mmH2O and 10mm H2O in the nasal cannula for triggering delivery of the medical gas.

12. A system according to any of claims 10 to 11, wherein the system furthermore is adapted for suggesting an amendment of the medical gas therapy applied and/or wherein the system is adapted for pro-actively adapting a medical gas therapy based on a patient's individual recorded previous breathing behaviour, or based on previously recorded breathing cycle information of a group of patients.

13. A system according to any of claims 10 to 12, wherein predicting a breathing behaviour comprises automatically adapting a medical gas therapy based on a patient's individual recorded previous breathing behaviour, or based on previously recorded breathing cycle information of a group of patients.

14. A system according to any of claims 10 to 13, wherein the system furthermore is configured for checking whether the used therapy differs from a predetermined prescription.

15. A medical gas container or an oxygenator comprising a system according to any of the previous claims.
